(19) **Europäisches Patentamt European Patent Office Office européen des brevets**

(11) **EP 4 596 829 A1**

(12) **EUROPEAN PATENT APPLICATION**
published in accordance with Art. 153(4) EPC

(43) Date of publication:
06.08.2025 Bulletin 2025/32

(21) Application number: 23930147.6

(22) Date of filing: 28.12.2023

(51) International Patent Classification (IPC):
*E21B 49/00* (2006.01)

(52) Cooperative Patent Classification (CPC):
**E21B 43/16; E21B 47/00; E21B 49/00**

(86) International application number:
**PCT/CN2023/142652**

(87) International publication number:
**WO 2024/198584 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 29.03.2023 CN 202310323908

(71) Applicant: PetroChina Company Limited
**Dongcheng District**
**Beijing 100007 (CN)**

(72) Inventors:
- DOU, Lirong
  **Beijing 100083 (CN)**
- LI, Xiangling
  **Beijing 100083 (CN)**
- XIAO, Kang
  **Beijing 100083 (CN)**
- LI, Xianbing
  **Beijing 100083 (CN)**
- XIAO, Kunye
  **Beijing 100083 (CN)**

(74) Representative: Ström & Gulliksson AB
**P.O. Box 4188**
**203 13 Malmö (SE)**

(54) **HIGH-DIP-ANGLE HETEROGENEOUS SANDSTONE RESERVOIR BIDIRECTIONAL FLOODING SIMULATION DEVICE AND METHOD**

(57) A high-dip-angle heterogeneous sandstone reservoir bidirectional flooding simulation device and method, relating to the field of oil and gas reservoir development. The high-dip-angle heterogeneous sandstone reservoir bidirectional flooding simulation device comprises a sand filling cavity (1); a plurality of layering indicator plates (3) are arranged in the sand filling cavity (1); the layering indicator plates (3) divide the sand filling cavity (1) into a plurality of sand filling modules, and the sand filling modules are filled with sandstones with different particle sizes; the layering indicator plates (3) each have one end connected to the side wall of the sand filling cavity (1), and are laid flat in the sand filling cavity (1); and the width of the layering indicator plates (3) is smaller than the width of the sand filling cavity (1), to ensure the fluid migration rule between two sand filling modules. In the high-dip-angle heterogeneous sandstone reservoir bidirectional flooding simulation device, the layering indicator plates (3) are designed, layering is carried out in the sand filling process according to indicators, and differentiated sand filling is carried out by using porous medium materials having different meshes, thereby meeting heterogeneous requirement of a model.

FIG. 1

## Description

### Field of the Invention

The present invention relates to the technical field of oil-gas reservoir development, in particular to a simulation device for bidirectional flooding of a heterogeneous sandstone reservoir with a high dip angle and a simulation method for bidirectional flooding of a heterogeneous sandstone reservoir with a high dip angle.

### Background of the Invention

The resources of heterogeneous sandstone reservoirs with a high dip angle are widely distributed, and the oil-water seepage law of the reservoirs with a high dip angle is greatly affected by gravity during the development of water flooding, making it difficult for crude oil production at the top position. A large amount of residual oil is formed at high positions and corners of the reservoirs, and the recovery rate of water flooding is low. Adopting a displacement mode of top gas injection and bottom water injection and making full use of the geological features of the high dip angle can effectively increase the production degree of such reservoirs, further increasing the development potential of the reservoirs. However, the heterogeneous characteristics of sandstone reservoirs with a high dip angle result in different fluid seepage laws in different regions, while the large dip angle of a formation also exacerbates the complexity of reservoir seepage during displacement, causing difficulties in understanding the mechanism of crude oil production.

At present, the conventional sand filling pipe device for simulating a core of the formation is a single-layer straight cylinder with both ends sealed, which can only simulate a core of a single formation, but cannot study the influence of heterogeneity on seepage, and cannot visually observe the fluid seepage state inside the sand filling pipe in different time periods and analyze the distribution law of residual oil. In the study of the sandstone reservoirs with a high dip angle, it is difficult for conventional simulation devices to reflect dip angle characteristics, and the simulation process cannot reflect the bidirectional displacement seepage law.

### Summary of the Invention

An object of the embodiments of the present invention is to provide a simulation device for bidirectional flooding of a heterogeneous sandstone reservoir with a high dip angle and a simulation method for bidirectional flooding of a heterogeneous sandstone reservoir with a high dip angle. The simulation device for bidirectional flooding of a heterogeneous sandstone reservoir with a high dip angle can achieve modular filling of sandstones to form different compositions, and simulate different heterogeneous sandstone reservoir structures, and simulation conditions are closer to those of actual reservoirs.

In order to achieve the above object, in a first aspect, an embodiment of the present invention provides a simulation device for bidirectional flooding of a heterogeneous sandstone reservoir with a high dip angle, including a sand filling cavity, wherein a plurality of layering indicator plates are disposed inside the sand filling cavity, the layering indicator plates divide the sand filling cavity into a plurality of sand filling modules, and each sand filling module is filled with sandstones with different particle sizes; and one ends of the layering indicator plates are connected to a side wall of the sand filling cavity, and the layering indicator plates are laid flat inside the sand filling cavity, and a width of each layering indicator plate is smaller than a width of the sand filling cavity for ensuring a fluid migration law between two sand filling modules.

Preferably, the simulation device further includes a compaction module including a compaction plate, a screw and a motor;

> a length of each layering indicator plate is less than a length of the sand filling cavity such that a compaction area is formed within the sand filling cavity, and the compaction area is located at an end of the sand filling cavity; and
> the compaction plate is placed inside the compaction area, the screw is connected with the compaction plate, and the motor drives the screw to move so that the compaction plate moves from the end to a center of the sand filling cavity to compact the sandstones inside the sand filling cavity.

Preferably, the simulation device further includes a sealing module including a first sealing cover and a second sealing cover which are separately disposed at both ends of the sand filling cavity for sealing the sand filling cavity; the screw passes through the second sealing cover and is threadedly connected with the second sealing cover, one end of the screw is placed inside the compaction area and is connected with the compaction plate, and the other end of the screw is placed outside the second sealing cover and is connected with an output shaft of the motor.

Preferably, the first sealing cover includes a first pressure-resistant sealing ring, and a first sealing block and a first sealing housing which are integrally disposed, the first sealing block is placed inside the sand filling cavity, the first sealing housing is connected with a housing of the sand filling cavity, and the first sealing cover and the sand filling cavity are sealed by the first pressure-resistant sealing ring; and

the second sealing cover includes a second pressure-resistant sealing ring, and a second sealing block and a second sealing housing which are integrally disposed, the second sealing block is placed inside the sand filling cavity, the second sealing housing is connected with the housing of the sand filling cavity, and the second sealing cover and the sand filling cavity are sealed by the second pressure-resistant sealing ring; and the second sealing cover is provided with a threaded hole through which the screw is sealingly connected with the second sealing cover.

Preferably, the simulation device further includes a high injection-production channel and a low injection-production channel which communicate with the sand filling cavity, the high injection-production channel is located in the first sealing cover, and the low injection-production channel is located in the second sealing cover.

Preferably, the simulation device further includes a plurality of waist injection-production channels communicating with the sand filling cavity, and the waist injection-production channels are uniformly disposed in the side wall of the sand filling cavity to monitor fluid distribution in the entire sand filling cavity.

Preferably, the simulation device further includes micro-flow samplers connected to injection-production channels through which the micro-flow samplers perform sampling.

Preferably, the simulation device further includes a variable dip angle module, which causes the sand filling cavity to dip according to a preset dip angle.

Preferably, the sand filling cavity is provided with a visualization window equipped with pressure-resistant and wear-resistant glass.

In a second aspect, an embodiment of the present invention provides a simulation method for bidirectional flooding of a heterogeneous sandstone reservoir with a high dip angle, implemented by the simulation device for bidirectional flooding of a heterogeneous sandstone reservoir with a high dip angle as described above, wherein the method includes the steps of:

test preparation: installing the simulation device for bidirectional flooding of a heterogeneous sandstone reservoir with a high dip angle, and performing test preparation on the simulation device for bidirectional flooding of a heterogeneous sandstone reservoir with a high dip angle, wherein the test preparation includes modular filling of a sand filling cavity, vacuum treatment of the sand filling cavity, and saturated fluid treatment of the sand filling cavity;

bidirectional displacement: injecting gas into a high position of a simulation device for a heterogeneous sandstone reservoir with a high dip angle and injecting water into a low position of the simulation device for a heterogeneous sandstone reservoir with a high dip angle, and producing oil from a waist of the simulation device for a heterogeneous sandstone reservoir with a high dip angle, forming bidirectional flooding development until the water content of a liquid produced from the waist reaches a preset water content, and ending the displacement;

analyzing production parameters of the liquid produced from the waist, and observing migration laws of an oil-gas interface and an oil-water interface, and a distribution law of residual oil during the bidirectional flooding; and

collecting a fluid microsample, analyzing the composition of the fluid microsample, and determining the characteristics of fluid migration.

Preferably, the method further includes: selecting a primary driving force of the reservoir based on geological information of the reservoir;

determining a driving force similarity criterion of the simulation device for bidirectional flooding of a heterogeneous sandstone reservoir with a high dip angle based on the selected primary driving force of the reservoir; and

designing driving force parameters of the simulation device for bidirectional flooding of a heterogeneous sandstone reservoir with a high dip angle based on the determined driving force similarity criterion.

In the simulation device for bidirectional flooding of a heterogeneous sandstone reservoir with a high dip angle, the layering indicator plates are designed, layering is performed during the sand filling process according to the prompts, and differentiated sand filling is performed by using porous medium materials with different mesh sizes to achieve the heterogeneous requirements of a model. The layering indicator plates achieve a modular design and controllable heterogeneity within the device. The monitoring requirements of gas phase migration dynamics and residual oil distribution at different development stages under high temperature and high pressure conditions are satisfied, and the visualization window assists in grasping the distribution characteristics and the migration process of crude oil, water, oil-water and oil-gas transition zones in the bidirectional displacement process of gas and water, and analyzing the production law of gas to residual oil.

Other features and advantages of the embodiments of the present invention will be described in detail in the subsequent Detailed Description.

## Brief Description of Drawings

The accompanying drawings are used to provide a further understanding of the embodiments of the present invention and constitute a part of this specification, and together with the detailed description below serve to explain, but not to limit, the embodiments of the present invention. In the accompanying drawings:

FIG. 1 is a cross-sectional view of a simulation device for bidirectional flooding of a heterogeneous sandstone reservoir with a high dip angle provided in Embodiment 1;

FIG. 2 is a front view of the simulation device for bidirectional flooding of a heterogeneous sandstone reservoir with a high dip angle provided in Embodiment 1;

FIG. 3 is a top view of the simulation device for bidirectional flooding of a heterogeneous sandstone reservoir with a high dip angle provided in Embodiment 1; and

FIG. 4 is a left view of the simulation device for bidirectional flooding of a heterogeneous sandstone reservoir with a high dip angle provided in Embodiment 1.

## Description of Reference Numerals

1-sand filling cavity, 2-cavity housing, 3-layering indicator plate, 4-compaction plate, 5-screw, 6-motor, 7-first sealing cover, 8-second sealing cover, 9-high injection-production channel, 10-low injection-production channel, 11-waist injection-production channel, 12-visualization window, 13-reinforcing rib plate, 14-variable dip angle shaft, 15-variable dip angle bracket, 16-hoop, 17-compaction area, and 18-first pressure-resistant sealing ring.

## Detailed Description of the Embodiments

A detailed description of the embodiments of the present invention will be described below with reference to the accompanying drawings. It should be understood that the specific embodiments described herein are merely illustrative and explanatory of the embodiments of the present invention, and are not intended to limit the embodiments of the present invention.

In the embodiments of the present invention, unless otherwise stated, directional words such as "up, down, left, and right" used generally refer to an orientation or positional relationship based on those shown in the drawings or an orientation or positional relationship in which the product of the present invention is conventionally placed when used.

The terms "first," "second," "third," and the like are used only to distinguish descriptions and cannot be understood as indicating or implying relative importance.

The terms "parallel", "perpendicular" and the like do not mean that the components are required to be absolutely parallel or perpendicular, but may be slightly inclined. For example, "parallel" merely means that its orientation is more parallel relative to "perpendicular" and does not mean that the structure must be perfectly parallel but may be slightly inclined.

The terms "horizontal," "vertical," "overhanging," and the like does not mean that the components are required to be absolutely horizontal, vertical, or overhanging, but may be slightly inclined. For example, "horizontal" merely means that its orientation is more horizontal relative to "vertical" and does not mean that the structure must be perfectly horizontal but may be slightly inclined.

In addition, the terms such as "approximately", "substantially" and the like are intended to indicate that the relevant content does not require absolute precision, but can have certain deviations. For example, "approximately equal" does not mean absolute equality only, and there is typically some variation due to the difficulty in achieving absolute equality during actual production and operation. Thus, "approximately equal" includes, in addition to being absolutely equal, the situations described above where there are some deviations. Taking this as an example, in other cases, unless otherwise specified, the terms "approximately", "substantially" and the like have meanings similar to those described above.

In the description of the present invention, it should also be noted that unless expressly specified and defined otherwise, the terms "disposed", "mounted", "connected", and "connection" should be understood in a broad sense, e.g., it may be fixedly connected, detachably connected, or integrally connected; it may be directly connected or indirectly connected through an intermediate medium, and it may be internal communication of two elements. The specific meaning of the above terms in the present invention will be understood by those of ordinary skill in the art according to specific situations.

"Connection" used herein is used to describe electrical power connection or signal connection between two components; and "connection" may be direct connection of two elements, connection through an intermediate medium (e.g., a wire), or indirect connection through a third element.

"Signal connection" used herein is used to describe signal connection between two components, such as a control signal and a feedback signal; the "electrical connection" is used to describe electrical power connection between two components; and "connection" may be direct connection between two parts or indirect connection through a third part.

Embodiment 1

A simulation device for bidirectional flooding of a heterogeneous sandstone reservoir with a high dip angle typically operates at a certain dip angle, and is divided into a high position and a bottom position. Bidirectional flooding refers to a reservoir development method that involves injecting water into a low position of the reservoir, injecting gas into the high position of the reservoir, and producing oil from a waist of the reservoir, forming water+gas bidirectional displacement.

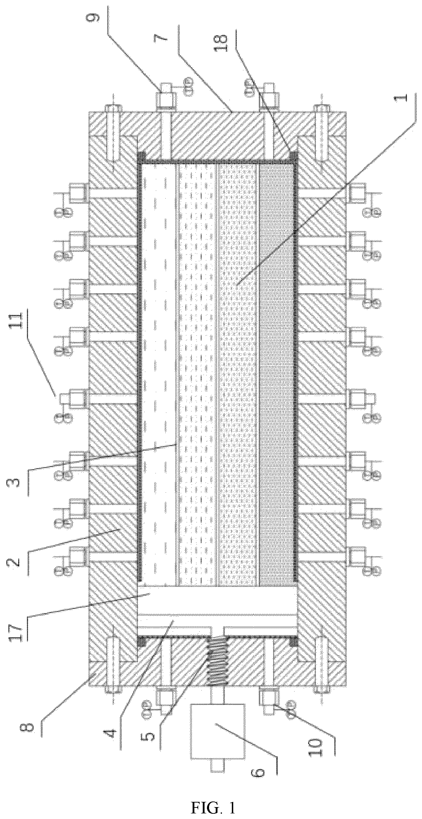

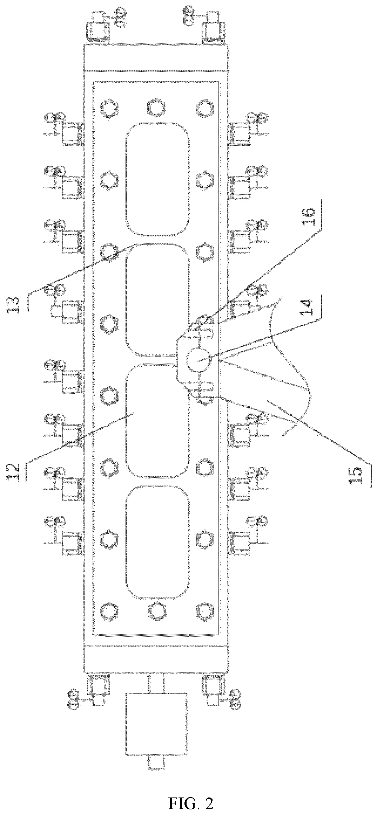

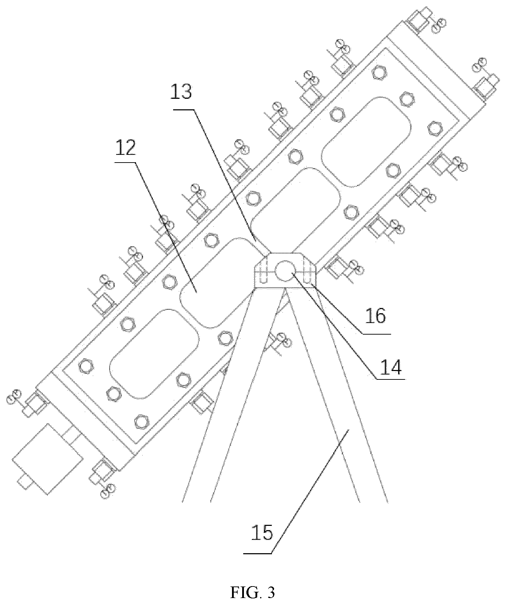

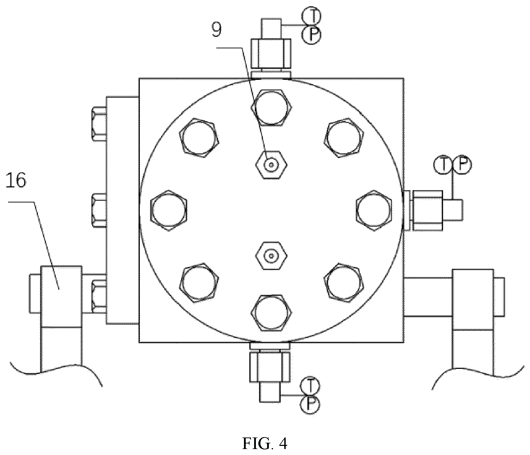

Referring to FIGS. 1-4, in a first aspect of this embodiment, provided is a simulation device for bidirectional flooding of a heterogeneous sandstone reservoir with a high dip angle, including a sand filling cavity 1, wherein a plurality of layering indicator plates 3 are disposed inside the sand filling cavity 1, and the layering indicator plates 3 divide the sand filling cavity 1 into a plurality of sand filling modules, and each sand filling module is filled with sandstones with different particle sizes; and one ends of the layering indicator plates 3 are connected to a side wall of the sand filling cavity 1, and the layering indicator plates 3 are laid flat inside the sand filling cavity 1, and a width of each layering indicator plate 3 is smaller than a width of the sand filling cavity 1 for ensuring a fluid migration law between two sand filling modules.

Specifically, the sand filling cavity 1 is surrounded by a cavity housing 2, which is a cuboid with a length of 80-120 cm, a width of 20-40 cm, and a thickness of 20-40 cm. The inside of the sand filling cavity 1 is used for sand filling to simulate a sandstone reservoir.

The layering indicator plates 3 are used to indicate layering when filling with the sandstones. The sandstones with different particle sizes are subjected to modular filling through the layering indicator plates 3. The sandstones with different particle sizes are subjected to filling in different combinations, which can simulate a variety of complex heterogeneous sandstone reservoirs.

Accordingly, each layering indicator plate 3 is a rectangular plate, a long side of each layering indicator plate 3 is parallel to a long side of the sand filling cavity 1, a wide side of each layering indicator plate 3 is parallel to a wide side of the sand filling cavity 1, one wide side wall of each layering indicator plate 3 is adjacent to an end face of the sand filling cavity 1, and the other wide side wall of the layering indicator plate 3 forms a compaction area 17 with the other end face of the sand filling cavity 1.

In this embodiment, each layering indicator plate 3 is a strip-shaped solid stainless steel plate having a thickness of 0. 1-0.5 cm and a width of 1-3 cm, and is fixed to an inner wall of a rear surface of the sand filling cavity housing by welding, and is used to indicate layering during sand filling, and at this time, sand filling materials with different mesh sizes are switched to achieve the heterogeneity characteristic of a sand filling model.

In other embodiments of the present invention, the layering indicator plates 3 are inclined at a certain angle to further form a complex simulated heterogeneous sandstone reservoir.

In this embodiment, the simulation device further includes a compaction module including a compaction plate 4, a screw 5 and a motor 6;

a length of each layering indicator plate 3 is less than a length of the sand filling cavity 1 such that a compaction area 17 is formed within the sand filling cavity 1, and the compaction area 17 is located at an end of the sand filling cavity 1; and the compaction plate 4 is placed inside the compaction area 17, the screw 5 is connected with the compaction plate 4, and the motor 6 drives the screw 5 to move so that the compaction plate 4 moves from the end to a center of the sand filling cavity 1 to compact the sandstones inside the sand filling cavity 1.

In this embodiment, the simulation device further includes a sealing module including a first sealing cover 7 and a second sealing cover 8 which are separately disposed at both ends of the sand filling cavity 1 for sealing the sand filling cavity 1; and the screw 5 passes through the second sealing cover 8 and is threadedly connected with the second sealing cover 8, one end of the screw 5 is placed inside the compaction area 17 and is connected with the compaction plate 4, and the other end of the screw 5 is placed outside the second sealing cover 8 and is connected with the output shaft of the motor 6. The sealing module is designed to resist a pressure of 50-80MPa.

In this embodiment, the first sealing cover 7 includes a first pressure-resistant sealing ring 18, and a first sealing block and a first sealing housing which are integrally disposed, the first sealing block is placed inside the sand filling cavity 1, the first sealing housing is connected with a housing of the sand filling cavity 1, and the first sealing cover 7 and the sand filling cavity 1 are sealed by the first pressure-resistant sealing ring 18; and the first sealing block is placed inside the sand filling cavity 1 and sealing is achieved in combination with the first pressure-resistant sealing ring 18.

The second sealing cover 8 includes a second pressure-resistant sealing ring, and a second sealing block and a second sealing housing which are integrally disposed, the second sealing block is placed inside the sand filling cavity 1, the second sealing housing is connected with the housing of the sand filling cavity 1, and the second sealing cover 8 and the sand filling cavity 1 are sealed by the second pressure-resistant sealing ring; and the second sealing cover 8 is provided with a threaded hole through which the screw 5 is sealingly connected with the second sealing cover 8.

Further, the second sealing cover 8 further includes a third pressure-resistant sealing ring coaxial with the screw 5, and the third pressure-resistant sealing ring is used to realize sealing between the screw 5 and the threaded hole.

In this embodiment, the simulation device further includes a high injection-production channel 9 and a low injection-production channel 10 which communicate with the sand filling cavity 1, the high injection-production channel 9 is located in the first sealing cover 7, and the low injection-production channel 10 is located in the second sealing cover 8.

In this embodiment, the high injection-production channel 9 includes a first high injection-production channel 9 and a second high injection-production channel 9, the low injection-production channel 10 includes a first low injection-production channel 10 and a second low injection-production channel 10, the first high injection-production channel 9 is located at an upper part of the first sealing cover 7, the second high injection-production channel 9 is located at a lower part of the first sealing cover 7, the first low injection-production channel 10 is located at an upper part of the second sealing cover 8, and the second low injection-production channel 10 is located at a lower part of the second sealing cover 8. The first high injection-production channel 9 and the second high injection-production channel 9 are distributed up and down, and the simultaneous injection-production effect is better when a simulation experiment is performed.

Further, the first high injection-production channel 9, the second high injection-production channel 9, the first low injection-production channel 10, and the second low injection-production channel 10 are each externally connected with a temperature collection point and a pressure collection point to test temperature data and pressure data of the injection-production channel.

Further, the first high injection-production channel 9, the second high injection-production channel 9, the first low injection-production channel 10, and the second low injection-production channel 10 are each externally connected with an injection-production pipeline and a micro-flow sampler; and the first high injection-production channel 9, the second high injection-production channel 9, the first low injection-production channel 10, and the second low injection-production channel 10 achieve water injection, gas injection, and oil production through the injection-production pipelines, and the first high injection-production channel 9, the second high injection-production channel 9, the first low injection-production channel 10, and the second low injection-production channel 10 achieve the acquisition of micro-flow fluids by the micro-flow samplers, reducing the effect of sampling on the internal seepage state of the device.

In this embodiment, the simulation device further includes a plurality of waist injection-production channels 11 communicating with the sand filling cavity 1, and the waist injection-production channels 11 are uniformly disposed in the side wall of the sand filling cavity 1 to monitor fluid distribution in the entire sand filling cavity 1.

Similarly, each waist injection-production channel 11 is also connected to an injection-production pipeline and a micro-flow sampler. Each waist injection-production channel 11 is externally connected with a temperature collection point and a pressure collection point.

The plurality of the waist injection-production channels 11 are distributed in the housing of the sand filling cavity 1 to facilitate monitoring the fluid distribution in the entire sand filling cavity 1.

In other embodiments of the present invention, the plurality of the waist injection-production channels 11 are distributed according to the actual sampling point requirements.

In this embodiment, the simulation device further includes micro-flow samplers connected to injection-production channels through which the micro-flow samplers perform sampling.

In this embodiment, the simulation device further includes a variable dip angle module, which causes the sand filling cavity 1 to dip according to a preset dip angle.

In this embodiment, the variable dip angle module consists of a variable dip angle shaft 14, a variable dip angle bracket 15 and a hoop 16. The variable dip angle shaft 14 is connected with the sand filling cavity 1 for controlling the sand filling cavity 1 to dip at a certain dip angle. The variable dip angle shaft 14 is connected to the variable dip angle bracket 15 by means of bearings. The variable dip angle shaft 14 is locked to a target dip angle by means of the hoop 16, and the variable dip angle module can achieve simulation of any angle from 0° to 90°.

In this embodiment, the sand filling cavity 1 is provided with a visualization window 12 equipped with pressure-resistant and wear-resistant glass. The visualization window 12 is fastened to the cavity housing 2 of the sand filling cavity 1 by bolts. The visualization window 12 is designed with a reinforcing rib plate 13. A visualization module is used to observe the distribution law of oil, gas and water inside the sand filling cavity 1. The pressure-resistant and wear-resistant glass is designed to resist a pressure of 50-80 MPa.

In a second aspect, an embodiment of the present invention provides a simulation method for bidirectional flooding of a heterogeneous sandstone reservoir with a high dip angle, implemented by the simulation device for bidirectional flooding of a heterogeneous sandstone reservoir with a high dip angle described above, wherein the method includes the steps:

test preparation: the simulation device for bidirectional flooding of a heterogeneous sandstone reservoir with a high dip angle is installed, and test preparation is performed on the simulation device for bidirectional flooding of a heterogeneous sandstone reservoir with a high dip angle, wherein the test preparation includes modular filling of a sand filling cavity 1, vacuum treatment of the sand filling cavity 1, and saturated fluid treatment of the sand filling cavity 1;

specifically, the simulation device for bidirectional flooding of a heterogeneous sandstone reservoir with a high dip angle is installed, a variable dip angle module is adjusted to a target dip angle, an external pipeline of a fluid collection system is connected, all valves are closed, a high injection-production channel 9 and a low injection-production channel 10 are opened, a high-pressure fluid is introduced into the high injection-production channel 9, the highest working pressure is maintained for 30-40 min, the pressure is built, and the qualified standard is no puncture or leakage.

A first sealing cover 7 at a high position is removed, and the sand filling cavity 1 is filled with a sand filling material such as quartz sand. After completion of sand filling, a sealing module is connected, and a motor 6 is turned on to push a compaction plate 4 to compact the sand body inside.

The low injection-production channel 10 is closed, and an external pipeline of the high injection-production channel 9 is connected to a vacuum pump for vacuuming; and

the high injection-production channel 9 is closed, an external pipeline of the low injection-production channel 10 is connected to a container containing water, and the water is saturated in a self-sucking manner. After completion of saturating the water, the high injection-production channel 9 is opened, and oil is injected into the high injection-production channel 9, and a material is discharged from the low injection-production channel 10. The fluid saturation is completed when the oil content of a liquid produced from the low injection-production channel 10 is 98% or more.

Bidirectional displacement: gas is injected into a high position of a simulation device for a heterogeneous sandstone reservoir with a high dip angle, water is injected into a low position of the simulation device for a heterogeneous sandstone reservoir with a high dip angle, and oil is produced from a waist of the simulation device for a heterogeneous sandstone reservoir with a high dip angle, forming bidirectional flooding development until the water content of a liquid produced from the waist reaches 98%, and the displacement ends;

production parameters of the liquid produced from the waist are analyzed, and migration laws of an oil-gas interface and an oil-water interface, and a distribution law of residual oil during the bidirectional flooding are observed; and a fluid microsample is collected, the composition of the fluid microsample is analyzed, and the characteristics of fluid migration are determined.

In this embodiment, the method further includes pressure relief and cleaning: the pressure of the simulation device for bidirectional flooding of a heterogeneous sandstone reservoir with a high dip angle is relieved, oil-containing waste sand in the sand filling cavity 1 is discharged, and fresh water is injected to clean the sand filling cavity 1 and the injection-production channels.

In this embodiment, the method further includes:

selecting a primary driving force of the reservoir based on geological information of the reservoir;
determining a driving force similarity criterion of the simulation device for bidirectional flooding of a heterogeneous sandstone reservoir with a high dip angle based on the selected primary driving force of the reservoir; and
designing driving force parameters of the simulation device for bidirectional flooding of a heterogeneous sandstone reservoir with a high dip angle based on the determined driving force similarity criterion.

Further, the method further includes:

determining a geometric similarity criterion and a physical similarity criterion of the simulation device for bidirectional flooding of a heterogeneous sandstone reservoir with a high dip angle; and
designing driving force parameters, geometric parameters, and physical parameters of the simulation device for bidirectional flooding of a heterogeneous sandstone reservoir with a high dip angle based on the determined driving force similarity criterion, geometric similarity criterion, and physical similarity criterion.

In particular, selecting the primary driving force of the reservoir based on the geological information of the reservoir is as follows: based on the analysis of driving energy, a driving mechanism of the reservoir is further subdivided into three driving forces, namely a gravity, a viscous force and a capillary force. Different mechanical conditions mainly depend on a displacement speed, a density difference, a mobility ratio difference, permeability, the capillary force, etc. The development characteristics of greater vertical permeability, a greater reservoir thickness, a greater difference in two-phase density, a lower capillary force, a small fluid viscosity, and a low development speed of the reservoir with a high dip angle are clarified, and the gravity number in a gravity dominant mode is determined as a main mechanical criterion.

Criteria for judging the primary driving force of the reservoir are as follows:
the gravity number and the capillary number are analyzed, and the gravity number is $N_G$. According to the formula, the physical meaning characterized by the gravity number is a ratio of a velocity caused by a gravity in a longitudinal direction to a velocity caused by a viscous force (a displacement pressure) in a horizontal direction, i.e. the relative magnitude of the gravity and the viscous force. In addition, the gravity number is also related to the shape of the reservoir.

The capillary number Nc characterizes the relative magnitude of the capillary force and the viscous force. The gas-oil mobility ratio is further considered on the basis of a dimensionless criterion to characterize the influence caused by a viscosity change of two phases of the gas and oil.

$$N_G = \frac{\Delta \rho g H_s \sin \alpha k_x}{\mu_o v_t L_s} = \frac{\frac{\Delta \rho g \sin \alpha k_x}{\mu_o}}{v_t} \cdot \frac{H_s}{L_s} \; ;$$

$$N_C = \frac{\sigma \cos \theta k_s}{\mu_o v_t L_S} \sqrt{\frac{\varphi}{k_s}} = \frac{\frac{\sigma \cos \theta k_s \sqrt{\frac{\varphi}{k_s}}}{\mu_o H_s}}{v_t} \cdot \frac{H_s}{L_S} \; ;$$

The two-phase flow of the reservoir, in addition to being influenced by the relative magnitude of the mechanical parameters, is also related to the properties of a displacement fluid, thus introducing the mobility ratio relationship into the dominant conditions for co-evaluation development.

According to the physical meaning of the formula, the boundaries of the different mechanical dominant conditions are shown in Table 1. Based on the established mechanical similarity criteria and the corresponding dominant conditions, corresponding physical experimental simulations and numerical simulations can be carried out, and the resulting laws can be applied to a mine site to actually evaluate the effect of this development method on improving the recovery ratio.

Table 1 Criterion boundaries for different mechanical dominant states

| Mechanical dominant state | Dominant criterion boundary |
| --- | --- |
| Viscous force dominance | $\dfrac{M(N_c + N_G)}{1 + M} < 1.0$ |
| Capillary force dominance | $\dfrac{MN_c}{1 + M} > 1.0$ |
| Gravity dominance | $\dfrac{MN_G}{1 + M} > 1.0$ |

Designing the driving force parameters, geometric parameters and physical parameters of the simulation device for bidirectional flooding of a heterogeneous sandstone reservoir with a high dip angle based on the determined driving force similarity criterion, geometric similarity criterion and physical similarity criterion is specifically as follows:

in a gravity dominant case, three similarity criteria are determined, including geometric similarity, physical similarity, and mechanical similarity.

$$\eta_1 = \frac{L_R}{L_m} \; ,$$

$$\eta_2 = \beta \frac{h_R}{h_m},$$

$$\eta_3 = \frac{\theta_R}{\theta_m} = 1,$$

$$\eta_4 = \gamma \frac{K_{wro} \cdot h_m \cdot \rho_o \cdot g \cdot h_m}{q_w \mu_w},$$

$$\eta_5 = \gamma \frac{K_{gro} \cdot h_m \cdot \rho_o \cdot g \cdot h_m}{q_g \mu_g},$$

$$\eta_6 = \frac{Q}{t},$$

$$\eta_7 = \frac{(\rho_o - \rho_g)gh\sin\theta_m K}{\mu_o v_{og} L_m},$$

$$\eta_8 = \frac{(\rho_w - \rho_g)gh\sin\theta_m K}{\mu_o v_{ow} L_m};$$

wherein $\eta_1$ - $\eta_3$ is a geometrical similarity criterion, $\eta_1$ is a dimensional similarity criterion, $\eta_2$ is a thickness similarity criterion, $\eta_3$ is a dip angle similarity criterion, $\eta_4$ - $\eta_6$ is a physical similarity criterion, $\eta_4$ and $\eta_5$, are flow rate similarity criteria, $\eta_6$ is a production time similarity criterion, $\eta_7$ - $\eta_8$ is a primary driving force similarity criterion, $\eta_7$ is a top air driving force similarity criterion, and $\eta_8$ is a bottom water driving force similarity criterion.

$L_R$: Length of the reservoir, m;

$L_m$: Length of the model, m;

$h_R$: Thickness of the reservoir, m;

$h_m$: Thickness of the model, m;

$\theta_R$: Dip angle of the reservoir, °;

$\theta_m$: Dip angle of the model, °;

$K_{wro}$: Water phase permeability under residual oil condition, mD;

$K_{gro}$: Gas phase permeability under residual oil condition, mD;

$\rho_o$: Oil phase density, kg/m$^3$;

$\rho_w$: Water phase density, kg/m$^3$;

$\rho_g$: Gas phase density, kg/m$^3$;

$q_w$: Water injection rate of model, m$^3$/s;

$q_g$: Gas injection rate of model, m$^3$/s;

Q: Injection volume, m$^3$;

$v_{og}$: Migration velocity of oil-gas interface, m/s;

$v_{ow}$: Migration velocity of oil-water interface, m/s;

$\mu_o$: Oil phase viscosity, mpa·s;

$\mu_w$: Water phase viscosity, mpa·s;
$\mu_g$: Gas phase viscosity, mpa·s; and
K: Permeability, mD.

According to the actual parameters of the reservoir, the parameters of the model and the development parameters during simulation are obtained by conversion. The emphasis is to introduce a scale factor, the thickness of the model weakens the effect of the thickness of the model by a thickness factor, and a gravity factor $\gamma$ is introduced to strengthen the influence of gravity under the condition of gravity dominance. When the gravity number is calculated, the effects of the gravity number and the gravity are further strengthened by designing the flow rate to influence the seepage velocity.

In the simulation device for bidirectional flooding of a heterogeneous sandstone reservoir with a high dip angle according to the present invention, the plurality of the waist injection-production channels 11 are designed. Sampling points are fully covered, and a bidirectional flooding development mode of high gas injection, bottom water injection and waist oil production is formed according to the demand, giving full play to the advantages of gravity differentiation of bidirectional flooding development in sandstone reservoirs with a high dip angle, and high efficiency of "water+gas" development. Different injection-production points can also be connected to micro-flow samplers or injection-production pipelines according to the demand. Sampling pipelines meet the monitoring requirements of gas phase migration dynamics and residual oil distribution at different development stages under high temperature and high pressure conditions.

The visualization window 12 is designed in the simulation device for bidirectional flooding of a heterogeneous sandstone reservoir with a high dip angle according to the present invention. The distribution characteristics and the migration process of crude oil, water, oil-water and oil-gas transition zones in the bidirectional displacement process of gas and water are monitored, and the production law of the gas to the residual oil is analyzed.

In the simulation device for bidirectional flooding of a heterogeneous sandstone reservoir with a high dip angle according to the present invention, the variable dip angle module is designed. Stable displacement at any angle of 0-90° is achieved by externally connecting with the variable dip angle bracket, satisfying the high dip angle conditions required for simulation.

In the simulation device for bidirectional flooding of a heterogeneous sandstone reservoir with a high dip angle according to the present invention, the layering indicator plates 3 are designed, and layering is performed during the sand filling process according to the prompts, and differentiated sand filling is performed by using porous medium materials with different mesh sizes to achieve the heterogeneous requirements of the model.

The layering indicator plates 3 achieve a modular design and controllable heterogeneity within the device. The monitoring requirements of gas phase migration dynamics and residual oil distribution at different development stages under high temperature and high pressure conditions are satisfied, and the visualization window 12 assists in grasping the distribution characteristics and the migration process of crude oil, water, oil-water and oil-gas transition zones in the bidirectional displacement process of gas and water, and analyzing the utilization law of the gas to the residual oil.

This embodiment provides the following various embodiments of different injection-production channels:

Case 1: when bottom water flooding simulation is performed at a certain dip angle, the low injection-production channel 10 is used as an injection port for water injection to simulate a water injection well, bottom water is formed at the bottom of the sand filling cavity 1, and the waist injection-production channels 11 and the high injection-production channel 9 are used as production ports to simulate an oil production well. The remaining injection-production channels are connected to micro-flow samplers for sampling and monitoring of the fluid distribution within the device. The effect of reservoir heterogeneity on bottom water flooding under the influence of the dip angle is explored.

Case 2: when gas cap flooding simulation is performed at a certain dip angle, the high injection-production channel 9 is used as an injection port to simulate a gas injection well, a gas cap is formed at the top of the sand filling cavity 1, and the waist injection-production channels 11 and the low injection-production channel 10 are used as production ports to simulate an oil production well. The remaining injection-production channels are connected to micro-flow samplers for sampling and monitoring of the fluid distribution within the device. The effect of reservoir heterogeneity on gas cap flooding under the influence of the dip angle is explored.

Case 3: when bidirectional flooding simulation is performed at a certain dip angle, the low injection-production channel 10 is used as an injection port for water injection to simulate a water injection well, and bottom water is formed at the bottom of the sand filling cavity 1; the high injection-production channel 9 is used as an injection port for gas injection to simulate a gas injection well, and a gas cap is formed at the top of the sand filling cavity 1. The waist injection-production channels 11 are used as production ports to simulate an oil production well. The remaining injection-production channels are connected to micro-flow samplers for sampling and monitoring of the fluid distribution within the device. The effect of reservoir heterogeneity on the bidirectional flooding under the influence of the dip angle is explored.

Case 4: water flooding is performed at a certain dip angle, the waist injection-production channels 11 are used as injection ports for water injection to simulate a water injection well, and the low injection-production channel 10 and the high injection-production channel 9 are used as production ports to simulate an oil production well. The remaining injection-production channel channels are connected to micro-flow samplers for sampling and monitoring of the fluid distribution within the device. The effect of reservoir heterogeneity on flooding effects of high and low positions of water displacement under the influence of the dip angle is explored.

This embodiment provides the following embodiments of two heterogeneous sandstone reservoirs composed of sandstones different particle sizes:

Case 1: when the target sand filling simulation effect is a low permeability positive rhythm reservoir, four kinds of quartz sands with a particle size of 20-40 meshes, 40-80 meshes, 80-100 meshes, and 100-120 meshes with good sorting and roundness are selected, and the four kinds of quartz sands are sequentially filled into the sand filling cavity in the order of particle sizes from large to small, and compacted and sealed.

Case 2: when the target sand filling simulation effect is a reverse rhythmic reservoir with a permeability difference of about 2, four kinds of quartz sands with a particle size of 30 meshes, 40 meshes, 50 meshes, and 60 meshes with good sorting and roundness are selected, and the four kinds of quartz sands are sequentially filled into the sand filling cavity in the order of particle sizes from small to large, and compacted and sealed.

Optional implementations of the embodiments of the present invention are described in detail above with reference to the accompanying drawings. However, the embodiments of the present invention are not limited to the specific details of the embodiments described above, and many simple variations can be made to the technical solutions of the embodiments of the present invention within the scope of the technical idea of the embodiments of the present invention, and these simple variations all fall within the protection scope of the embodiments of the present invention.

In addition, it should be noted that the specific technical features described in the above detailed description can be combined in any suitable manner without contradiction. In order to avoid unnecessary repetition, various possible combinations will not be described separately in the embodiments of the present invention.

Those skilled in the art will understand that implementing all or part of the steps of the method in the embodiments described above can be completed by instructing related hardware by a program, the program is stored in a storage medium and includes a plurality of instructions for causing a single chip microcomputer, a chip or a processor to execute all or part of the steps of the method according to the embodiments of the present application. The aforementioned storage medium includes various media that can store program codes, such as a USB flash disk, a mobile hard disk, a Read-Only Memory (ROM), a Random Access Memory (RAM), a magnetic disk, or an optical disk.

In addition, any combination between the various implementations of the embodiments of the present invention may be made, as long as they do not depart from the idea of the embodiments of the present invention, they should also be regarded as the contents disclosed in the embodiments of the present invention.

**Claims**

1.   A simulation device for bidirectional flooding of a heterogeneous sandstone reservoir with a high dip angle, comprising a sand filling cavity (1), wherein a plurality of layering indicator plates (3) are disposed inside the sand filling cavity (1), the plurality of the layering indicator plates (3) divide the sand filling cavity (1) into a plurality of sand filling modules, and each sand filling module is filled with sandstones with different particle sizes; and one ends of the layering indicator plates (3) are connected to a side wall of the sand filling cavity (1), and the layering indicator plates (3) are laid flat inside the sand filling cavity (1), and a width of each layering indicator plate (3) is smaller than a width of the sand filling cavity (1) for ensuring a fluid migration law between two sand filling modules.

2.   The simulation device for bidirectional flooding of a heterogeneous sandstone reservoir with a high dip angle according to claim 1, wherein the simulation device further comprises a compaction module comprising a compaction plate (4), a screw (5) and a motor (6);

a length of each layering indicator plate (3) is less than a length of the sand filling cavity (1) such that a compaction area (17) is formed within the sand filling cavity (1), and the compaction area (17) is located at an end of the sand filling cavity (1); and
the compaction plate (4) is placed inside the compaction area (17), the screw (5) is connected with the compaction plate (4), and the motor (6) drives the screw (5) to move so that the compaction plate (4) moves from the end to a center of the sand filling cavity (1) to compact the sandstones inside the sand filling cavity (1).

3. The simulation device for bidirectional flooding of a heterogeneous sandstone reservoir with a high dip angle according to claim 2, wherein the simulation device further comprises a sealing module comprising a first sealing cover (7) and a second sealing cover (8) which are separately disposed at both ends of the sand filling cavity (1) for sealing the sand filling cavity (1); and the screw (5) passes through the second sealing cover (8) and is threadedly connected with the second sealing cover (8), one end of the screw (5) is placed inside the compaction area (17) and is connected with the compaction plate (4), and the other end of the screw (5) is placed outside the second sealing cover (8) and is connected with an output shaft of the motor (6).

4. The simulation device for bidirectional flooding of a heterogeneous sandstone reservoir with a high dip angle according to claim 3, wherein the first sealing cover (7) comprises a first pressure-resistant sealing ring (18), and a first sealing block and a first sealing housing which are integrally disposed, the first sealing block is placed inside the sand filling cavity (1), the first sealing housing is connected with a housing of the sand filling cavity (1), and the first sealing cover (7) and the sand filling cavity (1) are sealed by the first pressure-resistant sealing ring (18); and the second sealing cover (8) comprises a second pressure-resistant sealing ring, and a second sealing block and a second sealing housing which are integrally disposed, the second sealing block is placed inside the sand filling cavity (1), the second sealing housing is connected with the housing of the sand filling cavity (1), and the second sealing cover (8) and the sand filling cavity (1) are sealed by the second pressure-resistant sealing ring; and the second sealing cover (8) is provided with a threaded hole through which the screw (5) is sealingly connected with the second sealing cover (8).

5. The simulation device for bidirectional flooding of a heterogeneous sandstone reservoir with a high dip angle according to claim 3, wherein the simulation device further comprises a high injection-production channel (9) and a low injection-production channel (10) which communicate with the sand filling cavity (1), the high injection-production channel (9) is located in the first sealing cover (7), and the low injection-production channel (10) is located in the second sealing cover (8).

6. The simulation device for bidirectional flooding of a heterogeneous sandstone reservoir with a high dip angle according to claim 1, wherein the simulation device further comprises a plurality of waist injection-production channels (11) communicating with the sand filling cavity (1), and the waist injection-production channels (11) are uniformly disposed in the side wall of the sand filling cavity (1) to monitor fluid distribution in the entire sand filling cavity (1).

7. The simulation device for bidirectional flooding of a heterogeneous sandstone reservoir with a high dip angle according to claim 1, further comprising micro-flow samplers connected to injection-production channels through which the micro-flow samplers perform sampling.

8. The simulation device for bidirectional flooding of a heterogeneous sandstone reservoir with a high dip angle according to claim 1, wherein the simulation device further comprises a variable dip angle module, which causes the sand filling cavity (1) to dip according to a preset dip angle.

9. The simulation device for bidirectional flooding of a heterogeneous sandstone reservoir with a high dip angle according to claim 1, wherein the sand filling cavity (1) is provided with a visualization window (12) equipped with pressure-resistant and wear-resistant glass.

10. A simulation method for bidirectional flooding of a heterogeneous sandstone reservoir with a high dip angle, implemented by the simulation device for bidirectional flooding of a heterogeneous sandstone reservoir with a high dip angle according to any one of claims 1-9, the method comprises the steps of:

    test preparation: performing test preparation on the simulation device for bidirectional flooding of a heterogeneous sandstone reservoir with a high dip angle, wherein the test preparation comprises modular filling of a sand filling cavity, vacuum treatment of the sand filling cavity, and saturated fluid treatment of the sand filling cavity;
    bidirectional displacement: injecting gas into a high position of a simulation device for a heterogeneous sandstone reservoir with a high dip angle and injecting water into a low position of the simulation device for a heterogeneous sandstone reservoir with a high dip angle, and producing oil from a waist of the simulation device for a heterogeneous sandstone reservoir with a high dip angle, forming bidirectional flooding development until the water content of a liquid produced from the waist reaches a preset water content, and ending the displacement;
    analyzing production parameters of the liquid produced from the waist, and observing migration laws of an oil-gas interface and an oil-water interface, and a distribution law of residual oil during the bidirectional flooding; and collecting a fluid microsample, analyzing the composition of the fluid microsample, and determining the characteristics of fluid migration.

11. The simulation method for bidirectional flooding of a heterogeneous sandstone reservoir with a high dip angle according to claim 10, further comprising:

selecting a primary driving force of the reservoir based on geological information of the reservoir;
determining a driving force similarity criterion of the simulation device for bidirectional flooding of a heterogeneous sandstone reservoir with a high dip angle based on the selected primary driving force of the reservoir; and
designing driving force parameters of the simulation device for bidirectional flooding of a heterogeneous sandstone reservoir with a high dip angle based on the determined driving force similarity criterion.

FIG. 1

FIG. 2

13

12

16

14

15

FIG. 3

FIG. 4

## INTERNATIONAL SEARCH REPORT

| International application No. |
|---|
| **PCT/CN2023/142652** |

### A. CLASSIFICATION OF SUBJECT MATTER

E21B49/00(2006.01)i

According to International Patent Classification (IPC) or to both national classification and IPC

### B. FIELDS SEARCHED

Minimum documentation searched (classification system followed by classification symbols)

IPC:E21B49

Documentation searched other than minimum documentation to the extent that such documents are included in the fields searched

Electronic data base consulted during the international search (name of data base and, where practicable, search terms used)

CNTXT, CNKI, DWPI, ENTXT: 非均质, 砂岩, 油藏, 模拟, 腔体, 砂石, 板, heterogeneity, sand, oil, reservoir, simulate, cavity, sheet

### C. DOCUMENTS CONSIDERED TO BE RELEVANT

| Category* | Citation of document, with indication, where appropriate, of the relevant passages | Relevant to claim No. |
|---|---|---|
| A | CN 204960927 U (CHINA NATIONAL OFFSHORE OIL CORPORATION et al.) 13 January 2016 (2016-01-13) description, pages 2-3, and figures 1-3 | 1-11 |
| A | CN 105096719 A (CHINA NATIONAL OFFSHORE OIL CORPORATION et al.) 25 November 2015 (2015-11-25) entire document | 1-11 |
| A | CN 109184640 A (CHINA PETROLEUM & CHEMICAL CORPORATION et al.) 11 January 2019 (2019-01-11) entire document | 1-11 |
| A | CN 110067545 A (CHINA PETROLEUM & CHEMICAL CORPORATION et al.) 30 July 2019 (2019-07-30) entire document | 1-11 |
| A | RU 2656303 C1 (OTKRYTOE AKTSIONERNOE OBSHCHESTVO SURGUTNEFTEGAZ) 04 June 2018 (2018-06-04) entire document | 1-11 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **27 March 2024** | **11 April 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/142652** |

| C. | DOCUMENTS CONSIDERED TO BE RELEVANT | |
|---|---|---|
| Category* | Citation of document, with indication, where appropriate, of the relevant passages | Relevant to claim No. |
| A | US 2021312111 A1 (SAUDI ARABIAN OIL COMPANY) 07 October 2021 (2021-10-07)<br>entire document | 1-11 |

Form PCT/ISA/210 (second sheet) (July 2022)

**INTERNATIONAL SEARCH REPORT**
Information on patent family members

International application No.

**PCT/CN2023/142652**

| Patent document cited in search report | | | Publication date (day/month/year) | Patent family member(s) | Publication date (day/month/year) |
|---|---|---|---|---|---|
| CN | 204960927 | U | 13 January 2016 | None | |
| CN | 105096719 | A | 25 November 2015 | None | |
| CN | 109184640 | A | 11 January 2019 | None | |
| CN | 110067545 | A | 30 July 2019 | None | |
| RU | 2656303 | C1 | 04 June 2018 | None | |
| US | 2021312111 | A1 | 07 October 2021 | None | |

Form PCT/ISA/210 (patent family annex) (July 2022)